(19) Europäisches Patentamt
European Patent Office
Office européen des brevets

(11) **EP 2 389 927 A1**

(12) **EUROPEAN PATENT APPLICATION**

(43) Date of publication:
**30.11.2011 Bulletin 2011/48**

(51) Int Cl.:
***A61K 9/16*** (2006.01)   ***A61K 9/20*** (2006.01)
***A61K 31/135*** (2006.01)

(21) Application number: **10164418.5**

(22) Date of filing: **30.05.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Abdi Ibrahim Ilac Sanayi ve Ticaret
Anonim Sirketi
Istanbul (TR)**

(72) Inventors:
• **Farshi, Farhad
  34555, ISTANBUL (TR)**
• **Avci, Recep
  34555, ISTANBUL (TR)**
• **Soylemez, Serdar
  34555, ISTANBUL (TR)**
• **Dude, Udaya
  34555, ISTANBUL (TR)**

(54) **Pharmaceutical formulations of rasagiline**

(57)     This invention is related to dissolving of rasagiline in a solvent or mixtures thereof, and treating the drug solution onto pharmaceutical material to provide the content uniformity.

EP 2 389 927 A1

## Description

### Technical Field

This invention is related to dissolving of rasagiline or pharmaceutically acceptable salts thereof in a solvent or mixtures thereof, and treating the drug solution onto pharmaceutical material to provide the content uniformity.

### Background Art

A movement disorder is a neurological disturbance that involves one or more muscle or muscle groups. Movement disorder affects a significant portion of population, causing disability as well as stress. Movement disorders include Parkinson's disease, Huntington's chorea, progressive supranuclear palsy, Wilson's disease, Tourrette's syndrome, epilepsy, tardive dyskinesia, and various chronic tremor's, ties and dystonias.

Parkinson's disease (also known as Parkinson disease or PD) is the one of the most common neurodegenerative disorder. Neurodegenerative means the degeneration, or death, of cerebral neurons. It is clinically characterized by resting tremor, rigidity, postrural instability, and bradykinesia. It affects approximately 1% of the population aged over 60 years worldwide. The incidence of parkinson's disease increase with age and the cumulative lifetime risk of an individual developing the disease is about 1 in 40.

Levodopa, dopamine agonists (such as Rotigotine, Pramipexole, Bromocriptine, Ropinirol, Cabergoline, Pergolide, Amorphine and Lisuride), COMT inhibitors (e.g.Entacapone, Tolcapone), MAO-B inhibitors (Selegiline, Rasagiline), anticholinergics (Trihexyphenidyl, Benztropine) and other Parkinson medication(s) (Amantadine) are used to treat parkinson's disease. In addition, Parkinson's disease can be treated by deep brain stimulation, and neurorehabilitation. The medications will most likely be used to increase the amount of dopamine in the brain. Levodopa is the primary treatment for Parkinson's disease; but, its long-term use at high dosages cause motor complications that can be difficult to manage. Moreover; dopamine agonist aren't preferred for treatment of early stage of Parkinson's disease. Because, dopamine agonists have more side effects and don't control symptoms as well as levodopa. However, monoamine oxidase type B (MAO-B) inhibitors are used in the early stages of Parkinson's disease to treat very mild symptoms such as resting tremor and delay the need for levodopa. Also, they have minimal side effects when used alone, and better medications are available for this purpose. In people with advanced Parkinson's disease who are taking levodopa, Selegiline, Rasagiline may be added to levodopa treatment to reduce motor fluctuctions, increase the time of effect of the levodopa and decrease the amount of levodopa needed to control symptoms. Selegiline is the first selective MAO-B inhibitor, has been marketed in United States since June 1989. Nevertheless, selegiline has toxic metabolites such as amphetamine and methamphetamine. Rasagiline is second-generation propargylamine pharmacophere that selectively and irreversibly inhibits brain MAO-B and is specifically designed for the treatment of Parkinson's disease and various other conditions. Pharmacologically, rasagiline was found to be $\leq 10$ -fold more potent than selegiline and was not metabolized to amphetamine derivatives.

Rasagiline was firstly disclosed by EP 0436492 B (TEVA PHARMACEUTICAL INDUSTRIES LTD.) 10.07.1991 .Rasagiline (INN) is designated chemically as (R)-N-(prop-2-ynyl)-2, 3-dihydro-1H-inden-1-amine and has the molecular formula $C_{12}H_{13}N$ and relative molecular mass of 171.238 g/mol.

The original salt form of rasagiline which was primarily studied in the pharmaceutical and toxicological tests was rasagiline hydrochloride. Rasagiline hydrochloride was also disclosed in. Afterwards some stability problems were reported for rasagiline hydrochloride. Then the mesylate salt form of rasagiline was disclosed by EP 812190 B (TEVA PHARMACEUTICAL INDUSTRIES LTD.) 17.12.1997 Published EP application EP 812190 B (TEVA PHARMACEUTICAL INDUSTRIES LTD.) 17.12.1997 discloses appearance of different salts of rasagiline under destructive conditions. Of these salts, mesylate is preferable for good solubility and chemical stability.

The acid salt form commonly used is rasagiline mesylate (molecular formula $(C_{12}H_{13}N)$ $CH_4SO_3$; relative mass 267.34 g/mol). Rasagiline mesylate is a white to off-white powder. Rasagiline is a chiral compound with one chiral center.

Rasagiline mesylate is a highly soluble compound. Water solubility is 0.01 ml, at pH 7.4 and also soluble in ethanol. It is sparingly soluble in isopropanol .Rasagiline is marketed in many countries as its mesylate salt. It is approved under trademark Azilet® by the US Food and Drug Administration for the treatment of the signs and symptoms of idiopathic Parkinson's disease as initial monotherapy and as adjunct therapy to levodopa.

Certain solid pharmaceutical composition comprising Rasagiline and process for their preparation are known.

Published PCT application WO 95/11016 A (TEVA PHARMACEUTICAL INDUSTRIES LTD.) 27.04.1995 and **U.S**. **Patent** US 6126968 A (TEVA PHARMACEUTICAL INDUSTRIES LTD.) 10.04.1997 disclose pharmaceutical compositions comprising rasagiline or pharmaceutically acceptable salt thereof and at least one alcohol selected from a group consists of pentahydric and hexahydric alcohols.

Published PCT Application WO 2006/091657 A (TEVA PHARMACEUTICAL INDUSTRIES LTD.) 31.08.2006 discloses solid pharmaceutical formulation of rasagiline comprising an amount of the mixture of particles of a pharmaceutically acceptable salt of rasagiline, wherein more than 90% of the total amount by volume of rasagiline salt particles have a size of less than 250 microns. According to our invention, content uniformity of dosage form which can be independently ensured from particule size distribution of rasagiline.

Published PCT Application WO 2009/122301 A (ACTAVIS GROUP PTC. EHT.) 08.10.2009 discloses a process for the preparation of Rasagiline mesylate having a $D_{90}$ particle size of about 600 microns to about 1500 microns, wherein; a) providing a solution of Rasagiline mesylate in a solvent medium comprising an ester solvent and an alcoholic solvent; b) subjecting the solution of step (a) to gradual cooling to produce a cooled solution; c) optionally, seeding the solution obtained in step (b); and (d) crystallizing Rasagiline mesylate particles having a $D_{90}$ particle size of about 600 microns to about 1500 microns from the cooled solution. The particles of step (d) were further milled to obtain Rasagiline mesylate having a $D_{90}$ particle size of about 255 microns to about 1400 microns.

Published EP Application EP 18274099 A (TEVA PHARMACEUTICAL INDUSTRIES LTD.) 05.09.2007 discloses solid pharmaceutical composition comprising rasagiline or pharmaceutically acceptable salt of rasagiline, and particles having a non-filamentous microstructure of at least two sugar alcohols such as mannitol, xylitol, sorbitol, maltitol and lactitol. The said composition also comprises a supplemental sugar alcohol, a supplemental flow agent and a supplemental disintegrant.

The above prior art references, especially WO 2006/091657 A (TEVA PHARMACEUTICAL INDUSTRIES LTD.) 31.08.2006 and WO 2009/122301 A (ACTAVIS GROUP PTC. EHT) 08.10.2009 describe reducing particle size of rasagiline to obtain content uniformity in solid dosage form. Reducing of particle size is achieved by comminution or mechanical process such as cutting, chipping, crushing, grinding, milling, micronizing, and trituration. However, reduction of particle size of rasagiline does not affect dissolution and so that gastrointestinal absorption and bioavailability. Because, it is readily soluble in water (approximately 617 mg/ml at 25 °C). Furthermore, reducing particle size of rasagiline is not advantageous. The main effect of reducing the particle size is to increase the surface area of active agent, which means that increasing the exposure surface to the environment. Physochemical properties and stability of rasagiline teaches that rasagiline has a tendency to produce oxidative, alkaline and acid degradation products. The increasing surface area can cause increasing oxidative degradation of rasagiline.

Nevertheless, there is a need a process for preparing content uniformity of solid oral dosage form comprising Rasagiline or pharmaceutical acceptable salt or pharmaceutical acceptable solvate thereof without comminution or a mechanical process. The inventors of the present invention surprisingly found that content uniformity of solid dosage form is achieved by dissolving rasagiline in a suitable solvent mixtures of thereof, followed by granulation with intragranular ingredients, then mixed with extra-granular ingredients and compressed the desired dosage form without any degradation products.

## Summary of invention

It is an object of the present invention to provide an improved process for a solid pharmaceutical composition comprising Rasagiline or pharmaceutically acceptable salt or pharmaceutical acceptable solvate thereof as the active ingredient which can be prepared without comminution or mechanical process reducing particle size of active agent; wherein the active ingredient is dissolved in a solvent.

Another aspect of the present invention is that said solution, which includes active pharmaceutical ingredient, is treated onto a pharmaceutical material or mixtures of pharmaceutical materials. This treatment can be adhering, adsorbing , absorbing ,coating, mixtures thereof and the like. Pharmaceutical material means any kind of excipients, pellets, granules, active or non-active containing tablet cores or mixture thereof and the like.

Treating the drug solution with an excipient or mixtures of excipients are performed by intragranulation or extragranulation phases. Intra-granular ingredient(s) comprising a dry mix containing filler/diluent, disintegrants, binders, glidants, surfactant and optionally stabilizers including suitable buffers or anti-oxidants or a mixture of thereof. Intragranulation can be carried out either by spraying technology in a fluidization processor or in high mixture granulator to knead the mass to form granule. Optionally a binder solution can be used for intragranulation.

Further, through drying the granular, sizing the dried granules; adding to the extragranular excipient(s), compressing the desired dosage form without any degradation products; thereby the content uniformity of dosage form will be achieved.

Extragranular excipients may be comprised filler/diluent, disintegrant, binder, glidants, lubricants and optionally stabilizers.

Technical Problem

For active pharmaceutical ingredients, particle size is important to attain compressibility, proper content uniformity, bioavailability, compactness of the final dosage form. Proper content uniformity and release profiles are major concerns in the process development. Proper blending and prevention of segregation, especially when other techniques are not feasible, are required for a successful processing.

Most particularly for low dose drugs, active pharmaceutical ingredients' (API) particle size distribution is the most critical point impacting the uniformity of low dose solid dosage forms. In order to have a homogeneous distribution, API particle size can be reduced to below a certain size. For instance to have content uniformity of dosage forms for rasagiline, WO 2006/091657 A (TEVA PHARMACEUTICAL INDUSTRIES LTD.) 31.08.2006 delineates reducing of particle size and less than 250 micron particle size. If API has greater particles, preparing of rasagiline dosage form becomes problematic so as to obtain content uniformity.

However, small particle size may cause manufacturing problems, hinder the processibility, thereby bringing about unnecessary delays in production. Other potential problems are related to reduce particle size include poor flowability and agglomeration which may result in segregation and poor blend uniformity.

The purpose is to minimize the segregation potential by improving content uniformity across the granule particle size distribution, thereby improving content uniformity in the tablet.

Solution to Problem

In order to eliminate the content uniformity problem in case of using greater particles of active pharmaceutical ingredient it is invented that rasagiline or pharmaceutically acceptable salt thereof is treated onto the surface of an excipient or mixtures of excipients. This method does not require reducing of particle size of rasagiline. Even though greater particle size is used, content uniformity is properly provided. Thus by invented method, there is no need to be taken into account the particle size of rasagiline.

**Description of embodiments**

The formulation comprises an effective amount of rasagiline and/or pharmaceutically acceptable salt or solvate thereof. In the following description, any reference to the term "rasagiline" is intended to include the pharmaceutically acceptable salts or solvates thereof, and especially rasagiline mesylate.

According to this invention, average particle size of Rasagiline mesylate used in formulation can be greater or lower than 250 $\mu$m. The term "average particle size" as used herein refers to the volume mean diameter of particles. The diameter and volume mean diameter can be determined by laser light scattering using e.g. a Malvern-Mastersizer Apparatus MS 2000. Particle sizes are determined by measuring the angular distribution of laser light scattered by a homogenous suspension of particles.

According to this invention formulations can be prepared by intragranulation and extragranulation steps. Intragranular ingredient(s) can be selected from the group consisting of diluents, binders, disintegrants, fillers, lubricants, glidants, antioxidants, surface active agents, chelating agents, mixtures thereof and the like.

The term 'filler' and the term 'diluent' are herein used interchangeably. Fillers fill out the size of a composition, making it practical to produce and convenient for the consumer to use. Suitable filler/diluent includes, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, a-lactose, β-lactose, Tablettose®, various grades of Pharmatose®, Microtose® or Fast-Floc®), methylcellulose polymers such as, e.g., Methocel A®, Methocel A4C®, Methocel A 15C®, Metocel A4M®), hydroxyethylcellulose, hydroxypropylcellulose, L-hydroxypropylycellulose (low substituted), hydroxypropyl methylcellulose (HPMC) (e.g. Methocel E®, F and K, Metolose SH® of Shin-Etsu, grades of Methocel F® and Metolose 65 SH®, the 4,000, 15,000 and 100,000 cps grades of Methocel K®; and the 4,000, 15,000, 39,000 and 100,000 grades of Metolose 90 SH®), sodium carboxymethylcellulose, carboxymethylene, carboxymethylhydroxyethylcellulose and other cellulose derivatives, starches or modified starches (including potato starch, wheat starch, corn starch, rice starch, pregelatinized maize starch), magnesium carbonate, magnesium oxide, maltitol, maltodextrins, maltose, sorbitol, starch, sucrose, sugar, and xylitol, erythritol.

A binder is used to impart cohesive qualities to the solid dosage form and thus ensure that a tablet remains intact after compression. Examples of binders include, but not limited to, microcrystalline cellulose, hydroxymethyl cellulose, hydroxypropylcellulose, starch (including corn starch and pregelatinized starch), gelatin, sugars (including sucrose, glucose, dextrose, lactose, and sorbitol), waxes, polyethylene glycol, natural and synthetic gums (e.g.acacia, tragacanth sodium alginate, celluloses, and Veegum),and synthetic polymers such as polymetacrylates and polyvinylpyrrolidone (povidone), ethylcellulose, hydroxyethyl cellulose, polyethylene oxide, mixtures thereof and the like.

A disintegrant is a substance which helps the composition break up once ingested .Disintegrants are, but not limited to, cross linked polyvinylpyrolidone (crospovidone, polyplyplasdone XL®, kollidon CL®); starches such as maize starch and dried sodium starch glycolate; gums such as maize starch and dried sodium starch glycolate; gums such as alginic acid, sodium alginate, guar gum; croscarmellose sodium; cellulose products such as microcrystalline cellulose and its salts, microfine cellulose, low-substituted hydroxypropylcellulose, mixtures thereof and the like.

An antioxidant is a molecule capable of slowing or preventing the oxidation of other molecules. Suitable anti-

oxidants are, but not limited to, ascorbic acid, sodium metabisulphite, butylated hydroxy anisole, butylated hydroxytoluene, sodium ascorbate, propyl gallate, alpha tocopherol, mixtures thereof and the like.

The solid pharmaceutical compositions of the present invention can further comprise extragranular ingredient(s). Extragranular ingredient(s) can be selected from filler/diluent, disintegrant, binder, glidants and lubricants. More preferably extragranular excipients are lubricant and glidant.

The presence of a lubricant is particularly preferred when the composition is a tablet as lubricants to improve the tabletting process. Lubricants prevent composition ingredients from clumping together and from sticking to the tablet punches or capsule filling machine and improve flowability of the composition mixture. Lubricants are, but not limited to sodium oleate, sodium stearate, sodium benzoate, sodium stearate, sodium chloride, stearic acid, sodium stearyl fumarate, calcium stearate, magnesium stearate, magnesium lauryl sulfate, sodium stearyl fumarate, sucrose esters or fatty acid, zinc, polyethylene glycol, talc, mixtures thereof and the like.

Glidants improve the flowability of the composition. The composition may also comprise a glidant. Glidants are, but not limited to, colloidal silica, powdered cellulose, talc, tribasic calcium phosphate, mixtures thereof and the like.

One or more these additives can be selected and used by the artisan having regard to the particular desired properties of the solid dosage form. The amount of each type of additive employed, e.g. glidant, binder, disintegrant, filler or diluent and lubricant may vary within ranges conventional in the art.

Suitable pharmaceutical compositions include, but are not limited to, capsules, tablets, granules, powders and unit dose pockets. Preferably oral pharmaceutical formulation is tablet. The tablet can be coated or non-coated.

Pharmaceutical formulations of the present invention are prepared by dissolving rasagiline in a solution. Solution may include an aqueous solvent or a non-aqueous solvent of mixture thereof or the like.

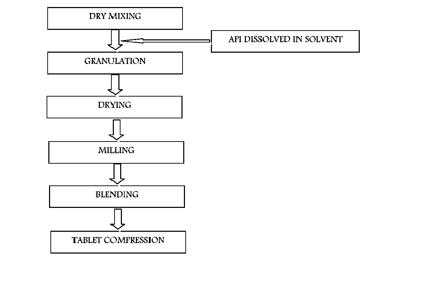

Pharmaceutical formulations can be prepared by using the process diagrammed in Figure 1.

In an embodiment of the present invention, a process for preparing content uniformity of solid pharmaceutical composition comprising intra-granular ingredient(s), extra-granular ingredient(s) and comprise the steps of:

**a. Intragranulation**

1. dissolving Rasagiline in a solvent to form drug solution,
2. Loading particles of the intra-granular ingredients into granulator and treating the drug solution of step (a) with particles of the intra-granular ingredients either by spraying technology in a fluidization processor or in high shear granulator.
3. kneading the mass of step (b) to form granules and optionally granulate with a suitable pharmaceutically binder solution,
4. Drying the granular mass of step (c) in fluidized bed dryer until proper moisture then sizing the dried granules by using suitable milling equipment.

**b.Extragranulation**

1. mixing extra-granular excipient(s)with the granules of step (d) and blending to form final blend,
2. Processing the final blend into a suitable dosage form which is suitable for oral administration.

In a further embodiment, Rasagiline mesylate dissolved in a pharmaceutically acceptable solvent selected from the group consisting of water, ethanol or Isopropyl alcohol or mixtures thereof and the like. Solution may include other pharmaceutical material(s).

In a further embodiment, solvents used for granulation process may be selected from water, isopropyl alcohol, acetone, ethanol, methylene chloride or combination thereof.

The present invention is illustrated by the following examples without being limited.

Intragranulation comprising active ingredient is from about 0.1 % to about 5%, filler/diluents is from about 30% to about 90% by the weight of composition, disintegrant is from about 3% to about 50% by weight of the composition, and binder is from about 3% to about 50% by weight of intragranular phase's ingredients.

Preferably, intragranulation comprising rasagiline mesylate is %0.78, filler/diluent is 79.30%, disintegrant is 9.96%, binder is 9.96% by total weight of intragranulation phase's ingredients. (Table 1).

**Table 1**

| Intragranulation | | |
|---|---|---|
| **Ingredients** | **mg** | **%** |
| Rasagiline Mesylate | 0.78 | 0.78% |
| Dissolving Solvent | Evaporated | 0.00% |
| Filler/Diluent | 79.62 | 79.30% |
| Disintegrant | 10.00 | 9.96% |
| Binder | 10.00 | 9.96% |
| Intragranular Total | 100.40 | 100% |

Extragranulation comprising glidant is from about 10% to about 90%, lubricant is from about 10% to about 90%by total weight of extragranular phase's excipients.

Preferably, extragranulation comprising glidant is 56.52%, lubricant is 43.48 % by total weight of extragranulation step's excipients (Table-2).

**Table 2**

| Extragranulation | | |
|---|---|---|
| **Ingredients** | **mg** | **%** |
| Glidant | 2.60 | 56.52% |
| Lubricant | 2.00 | 43.48% |
| Extragranul ar Total | 4.60 | **100.00%** |

In another aspect, pharmaceutical composition comprising intragranular and extragranular steps wherein intragranular total is from%15 to %99 , extragranular total is from%3 to % 85 by the weight of total composition. Preferably intragranular total is 95.62%, extragranular total is 4.38 % by weight of pharmaceutical composition. (Table-3).

**Table 3**

| Total Pharmaceutical Composition | | |
|---|---|---|
| | **mg** | **%** |
| Intragranular Total | 100.40 | 95.62% |
| Extragranular Total | 4.60 | 4.38% |
| **Total** | **105.00** | **100.00%** |

In total, pharmaceutical composition comprising rasagiline or pharmaceutically acceptable salt thereof is from about 0.1 % to about 5%, filler/diluent is from about 30% to about 90%, disintegrant is from about 3 % to about 20%, binder is from about 3 % to about 20%, glidant is from about 0.1 % to about 10%, lubricant is from about 0.1% to about 10 by weight of pharmaceutical composition.

In total, pharmaceutical composition preferably comprising rasagiline or pharmaceutically acceptable salt thereof is 0.74%, filler/diluent is 75.83 %, disintegrant is 9.53 %, binder is 9.53 %, glidant is 2.47%, lubricant is 1.90 % by weight of pharmaceutical composition (Table-4).

**Table 4**

| Total Pharmaceutical Composition | | |
|---|---|---|
| **Ingredients** | **mg** | **%** |
| Rasagiline Mesylate | 0.78 | 0.74 |
| Filler/Diluent | 79.62 | 75.83% |

(continued)

| Total Pharmaceutical Composition | | |
|---|---|---|
| **Ingredients** | **mg** | **%** |
| Binder | 10.00 | 9.53% |
| Disintegrant | 10.00 | 9.53% |
| Glidant | 2.60 | 2.47% |
| Lubricant | 2.00 | 1.90% |
| **Total** | **105.00** | **100.00%** |

In another aspect, the purpose of this invention is to minimize the segregation by improving content uniformity across the granule particle size distribution, thereby improving content uniformity in the tablet.

Although the EP (European Pharmacopeia) is not a regulatory body, their recommendations for testing protocols are readily adopted by pharmaceutical companies. In the case of content uniformity EP: 1. The actual % API in 10 tablets is tested and the acceptance value (AV) is calculated. The requirements for dosage uniformity are met if the acceptance value (AV) of the first 10 dosage units is less than or equal to L1 If the acceptance value is greater than L1, next 20 dosage units must be tested and the acceptance value must be calculated. The requirements are met if the final acceptance value of 30 dosage units is less than or equal to L1 and no individual content of the dosage unit is less than (1-L2*0.01)M or more than(1+L2*0.01)M in calculation of acceptance value under content uniformity or under mass variation. (L1 =15.0,L2=25.0 unless otherwise specified, ).2. The Relative Standard Deviation (RSD) is no greater than 6.0%.

**Table 5**

| Content Uniformity Result (%) | |
|---|---|
| Sample -1 | 100.6% |
| Sample -2 | 98.6% |
| Sample -3 | 102.2% |
| Sample -4 | 102.9% |
| Sample -5 | 101.4% |
| Sample -6 | 101.5% |
| Sample -7 | 100.9% |
| Sample -8 | 102.3% |
| Sample -9 | 104.8% |
| Sample -10 | 101.9% |
| Mean | 101.7% |
| SD | 1.609 |
| RSD% | 1.58 |
| AV | 4.062 |

The above results clearly show that the content uniformity is ensured. The RSD% is below the limit.

A suitable dissolution method is used to reach dissolution profiles. In this invention USP Type-2 apparatus is preferred and used. According to the method which was developed during drug development process, the tablets prepared in accordance with the present invention should exhibit the following dissolution profile when tested in a USP Type 2 apparatus, at 50 rpm, and 37° C and in 0.1 N HCl medium.

To determine the similarity between the dissolution profiles of the test and reference product a simple model independent approach, that is $f_2$ (similarity factor), was carried out. As per US FDA, $f_2$ values should lie between 50-100 for developing two similar dissolution profiles.

The similarity factor $f_2$ is a measurement of the similarity through a point by point comparison as shown in equation 1.

$$f_2 = 50 * \log \frac{100}{\sqrt{1 + \frac{1}{n}\sum_{t=1}^{n}(R_t - T_t)^2}}$$

Equation-1

n: is the number of sampling time points

Rt : is the amount drug released from a reference batch at time t

Tt : is the amount drug released from a test batch at time t.

Generally, f2 values greater than 50 ensure sameness of the performance of the reference product and test product.

The test product and the reference product's results show that the $f_2$ is in the limits of 50- 100 as established by the US FDA for claiming similarity between the dissolution profiles of the test and reference product.

**Claims**

1.  A preparation method of pharmaceutical composition of rasagiline or pharmaceutically acceptable salts thereof **characterized in that**: a-) rasagiline or pharmaceutically acceptable salts thereof and optionally an excipient or mixtures of excipients are dissolved in a solvent or mixtures of solvents and b-) said solution of rasagiline or pharmaceutically acceptable salts thereof are treated onto a pharmaceutical material or mixtures of pharmaceutical materials.

2.  As claimed in claim 1, a pharmaceutical composition of rasagiline or pharmaceutically acceptable salts thereof comprises filler/diluent, disintegrant, binder, glidant, lubricant and optionally an antioxidant.

3.  According to claim 1, a pharmaceutical composition, is prepared in at least two phases wherein -intragranular phase comprising the active is dissolved and treated onto intragranular ingredients - and extragranular phase comprising intragranular phase's ingredients, glidant and lubricant.

4.  According to preceding claim 3, intragranulation comprising : rasagiline or pharmaceutically acceptable salt is from %0.1 to %5, filler/diluents is from 30% to 95% ,disintegrant is from 3% to 50%, binder is from 3% to 50% by weight of intragranular phase's excipients.

5.  According to preceding claim 4, intragranular phase of pharmaceutical composition comprises following percentages by weight of total intragranular phase mixture: rasagiline or pharmaceutically acceptable salt is 0.78%, filler/diluents is 79.30%, disintegrant is 9.96%, binder is 9.96%.

6.  According to claim 3, extragranulation comprising: glidant is from 10% to 90%, lubricant is from 10% to 90% by total weight of extragranular phase's excipients.

7.  According to claim 6, extragranular phase of pharmaceutical composition comprises following percentages by weight of total extragranular phase mixture: glidant is 56.52%, lubricant is 43.48% .

8.  According to claim 1, a process for preparing pharmaceutical composition comprises the steps of: a)Rasagiline is dissolved in a solution, b)loading particles of the intra-granular ingredients into granulator machine and treat the drug solution of step (a) with particles of the intra-granular ingredients c)kneading the wet mass of step (b) to form granules and optionally granulate with a suitable pharmaceutically binder solution, d) drying the granular mass of step (c) e)mixing extra-granular excipient(s) with the granules of step (d) and blending to form final blend, f) processing the final blend into a suitable dosage form which is suitable for oral administration

9.  According to claim 1, the pharmaceutical composition, comprising:rasagiline or pharmaceutically acceptable salts thereof is in the range of from 0.1 % to 5%, filler/diluent is in the range of from 30% to 90%, disintegrant is in the range of from 3% to 20%, binder is from 3% to 20%, glidant is from 0.1 % to 10%,lubricant is from 0.1 % to 10% by weight of tablet core.

**10.** According to claim 9, pharmaceutical composition comprising rasagiline or pharmaceutically acceptable salts thereof is 0.74%, filler/diluent is 75.83 %, disintegrant is 9.53 %, binder is 9.53 %, glidant is 2.47%, lubricant is 1.90 % by weight of tablet core.

**11.** According to claim 1,the pharmaceutical composition provides content uniformity by having following properties: a) RSD$\leq$%6 and b)AV <L1

**12.** According to claim 1, the pharmaceutical composition has a similarity factor of (f2) over 50 when compared with reference tablet.

```
┌──────────────────────┐
│      DRY MIXING      │
└──────────────────────┘
           ⇩              ┌────────────────────────────┐
┌──────────────────────┐◄─┤  API DISSOLVED IN SOLVENT  │
│     GRANULATION      │  └────────────────────────────┘
└──────────────────────┘
           ⇩
┌──────────────────────┐
│       DRYING         │
└──────────────────────┘
           ⇩
┌──────────────────────┐
│       MILLING        │
└──────────────────────┘
           ⇩
┌──────────────────────┐
│      BLENDING        │
└──────────────────────┘
           ⇩
┌──────────────────────┐
│  TABLET COMPRESSION  │
└──────────────────────┘
```

Europäisches
Patentamt

European
Patent Office

Office européen
des brevets

## EUROPEAN SEARCH REPORT

Application Number

EP 10 16 4418

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2010/070090 A1 (RATIOPHARM GMBH [DE]; RIMKUS KATRIN [DE]; BRUECK SANDRA [DE]; MUSKULUS) 24 June 2010 (2010-06-24)<br>* page 9; example 9 *<br>* page 13; example 13 *<br>* page 14; example 14 *<br>* page 15; example 15 *<br>* page 16; example 16 *<br>* page 17; claims 9,10; example 17 *<br>----- | 1-12 | INV.<br>A61K9/16<br>A61K9/20<br>A61K31/135 |
| E | WO 2010/111264 A2 (REDDYS LAB LTD DR [IN]; REDDYS LAB INC DR [US]; GUPTA DEEPAK [IN]; NAG) 30 September 2010 (2010-09-30)<br>* pages 14,15; example 1 *<br>* page 17; example 4 *<br>* page 18; example 5 *<br>* pages 20,21; example 8 *<br>----- | 1-12 | |
| E | WO 2010/100219 A2 (SANDOZ AG [CH]; TALASILA KAMALAKAR [IN]; NARKHEDE HEMANT [IN]; DAROI A)<br>10 September 2010 (2010-09-10)<br>* page 6; example 1 *<br>----- | 1-12 | |
| X | US 4 489 026 A (YALKOWSKY SAMUEL H [US])<br>18 December 1984 (1984-12-18)<br>* column 1, lines 5-23 *<br>* column 2, lines 51-58 *<br>* column 3, lines 4-36 *<br>* column 3, line 54 - column 5, line 68; claims 1-7 *<br>-----<br>-/-- | 1-12 | |

TECHNICAL FIELDS
SEARCHED      (IPC)

A61K

The present search report has been drawn up for all claims

| Place of search | Date of completion of the search | Examiner |
|---|---|---|
| Munich | 30 November 2010 | Toulacis, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 10 16 4418

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/097076 A2 (SMITHKLINE BEECHAM CORP [US]; FAULKNER PATRICK [US]; PAN RENNAN [US];) 20 October 2005 (2005-10-20) <br> * page 1, lines 15-22 * <br> * page 2, line 28 - page 3, line 4 * <br> * page 3, lines 25-30 * <br> * page 3, line 39 - page 4, line 23 * <br> * pages 11-16; examples 1-4 * <br> ----- | 1-12 | |

TECHNICAL FIELDS
SEARCHED        (IPC)

The present search report has been drawn up for all claims

| Place of search | Date of completion of the search | Examiner |
|---|---|---|
| Munich | 30 November 2010 | Toulacis, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                    EP 10 16 4418

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2010

| Patent document cited in search report | | Publication date | Patent family member(s) | Publication date |
|---|---|---|---|---|
| WO 2010070090 | A1 | 24-06-2010 | DE 102008064061 A1 | 24-06-2010 |
| WO 2010111264 | A2 | 30-09-2010 | NONE | |
| WO 2010100219 | A2 | 10-09-2010 | NONE | |
| US 4489026 | A | 18-12-1984 | NONE | |
| WO 2005097076 | A2 | 20-10-2005 | EP 1734930 A2<br>JP 2007532511 T | 27-12-2006<br>15-11-2007 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0436492 B
- EP 812190 B
- WO 9511016 A
- US 6126968 A
- WO 2006091657 A
- WO 2009122301A A
- EP 18274099 A
- WO 2009122301 A